(19) **Europäisches Patentamt**
**European Patent Office**
**Office européen des brevets**

(11) **EP 1 404 880 B1**

(12) **EUROPÄISCHE PATENTSCHRIFT**

(45) Veröffentlichungstag und Bekanntmachung des Hinweises auf die Patenterteilung:
**20.01.2010 Patentblatt 2010/03**

(21) Anmeldenummer: **02782459.8**

(22) Anmeldetag: **05.07.2002**

(51) Int Cl.:
*C12Q 1/68* (2006.01)     *B01J 19/00* (2006.01)

(86) Internationale Anmeldenummer:
**PCT/EP2002/007539**

(87) Internationale Veröffentlichungsnummer:
**WO 2003/004699 (16.01.2003 Gazette 2003/03)**

(54) **VERFAHREN ZUM NACHWEIS VON IN EINER POLYMERASE-KETTENREAKTION AMPLIFIZIERTEN NUKLEINSÄUREMOLEKÜLEN**

METHOD FOR IDENTIFYING NUCLEIC ACID MOLECULES AMPLIFIED IN A POLYMERASE CHAIN REACTION

PROCEDE DE MISE EN EVIDENCE DE MOLECULES D'ACIDE NUCLEIQUE AMPLIFIEES DANS UNE REACTION EN CHAINE DE LA POLYMERASE

(84) Benannte Vertragsstaaten:
**AT BE BG CH CY CZ DE DK EE ES FI FR GB GR IE IT LI LU MC NL PT SE SK TR**

(30) Priorität: **06.07.2001 DE 10132785**

(43) Veröffentlichungstag der Anmeldung:
**07.04.2004 Patentblatt 2004/15**

(73) Patentinhaber: **CLONDIAG GmbH 07749 Jena (DE)**

(72) Erfinder:
• **SCHULZ, Torsten**
  **07743 Jena (DE)**
• **ERMANTRAUT, Eugen**
  **07745 Jena (DE)**
• **POSER, Siegfried**
  **07749 Jena (DE)**
• **KAISER, Thomas**
  **07743 Jena (DE)**
• **MÖBIUS, Klaus-Peter**
  **07751 Zöllnitz (DE)**
• **ULLRICH, Thomas**
  **07743 Jena (DE)**
• **ADELHELM, Karin**
  **07743 Jena (DE)**

(74) Vertreter: **Neuefeind, Regina Maiwald Patentanwalts GmbH Elisenhof Elisenstrasse 3 80335 München (DE)**

(56) Entgegenhaltungen:
WO-A-00/17401     WO-A-01/02094
WO-A-98/50581     WO-A-99/50446

• NILSSON P ET AL: "REAL-TIME MONITORING OF DNA MANIPULATIONS USING BIOSENSOR TECHNOLOGY" ANALYTICAL BIOCHEMISTRY, ACADEMIC PRESS, SAN DIEGO, CA, US, Bd. 224, Nr. 1, 1995, Seiten 400-408, XP000486750 ISSN: 0003-2697
• SOUTHERN E M: "DNA chips: analysing sequence by hybridization to oligonucleotides on a large scale" TRENDS IN GENETICS, ELSEVIER SCIENCE PUBLISHERS B.V. AMSTERDAM, NL, Bd. 12, Nr. 3, 1. März 1996 (1996-03-01), Seiten 110-115, XP004037238 ISSN: 0168-9525
• WATTS H J ET AL: "REAL-TIME DETECTION AND QUANTIFICATION OF DNA HYDRIDAZATION BY AN OPTICAL BIOSENSOR" ANALYTICAL CHEMISTRY, AMERICAN CHEMICAL SOCIETY. COLUMBUS, US, Bd. 67, Nr. 23, 1. Dezember 1995 (1995-12-01), Seiten 4283-4289, XP000540115 ISSN: 0003-2700
• KAI E. T AL: ANALYTICAL CHEMISTRY, Bd. 71, Nr. 4, 15. Februar 1999 (1999-02-15), Seiten 796-800, XP009004116

EP 1 404 880 B1

**Beschreibung**

Die Erfindung betrifft eine Vorrichtung sowie ein Verfahren zum qualitativen und/oder quantitativen Nachweis von in einer Polymerase-Kettenreaktion amplifizierten Nukleinsäuremolekülen.

Bei der Polymerase-Kettenreaktion (PCR, Polymerase Chain Reaction) wird durch repetitives Wiederholen eines Temperaturregimes ein DNA-Template exponentiell und sequenzspezifisch vervielfältigt (siehe u.a. US 4,683,195, US 4,683,202 , US 4,965,188, EP 0502588, M.J. McPherson, D.D. Hames, G.R. Taylor, PCR 1 und 2: A practical Approach, IRL Press at Oxford University Press, Oxford (1995)). Bei der Denaturierungstemperatur wird die zu amplifizierende DNA zu einzelsträngiger DNA aufgeschmolzen. Bei der folgenden niedrigeren Annealingtemperatur binden die Primer an die beiden DNA-Einzelstränge. Es folgt die etwas höhere Extensionstemperatur, bei der die beiden DNA-Einzelstränge durch eine Enzymkatalysierte Reaktion zu Doppelsträngen aufgefüllt werden. Der Zyklus wird nahezu beliebig oft wiederholt, bis die amplifizierte DNA, das PCR-Produkt, in analytisch verwertbarer Menge vorliegt. Die PCR ist eines der wichtigsten Verfahren in der DNA-Analytik und besitzt deswegen eine ausgesprochen hohe wirtschaftliche Bedeutung.

Ein wesentliches Problem bei der PCR stellt die Bestimmung der Anzahl an PCR-Zyklen dar, die zur Amplifikation einer gewünschten, ggf. analytisch verwertbaren Menge an Nukleinsäure erforderlich ist. Beispielsweise können zu diesem Zweck Farbstoffe eingesetzt werden, die sich in doppelsträngige DNA interkalieren oder an doppelsträngige DNA anlagern und somit das PCR-Produkt sichtbar machen.

In F. Lottspeich, H. Zorbas, 'Bioanalytik', Spektrum Akademischer Verlag, Heidelberg (1998), ist u.a. der Farbstoff SYBR Green beschrieben, der sich spezifisch - einem Interkalator vergleichbar - ausschließlich an doppelsträngiger DNA anlagert. Durch die Anlagerung wird der Farbstoff fluoreszenzaktiv. Der Farbstoff wird der PCR zugesetzt. Durch die Zunahme des gemessenen Fluoreszenzlichts bei der Extensionstemperatur wird die Bildung von doppelsträngiger DNA angezeigt. Ferner kann bei dieser Methode nach Beendigung der PCR durch Erhitzen der Probe auf 95°C die Temperatur bestimmt werden, bei der das Fluoreszenzlicht durch den DNA-Aufschmelzprozess verschwindet. Auf diese Weise kann auf den GC-Gehalt eines PCR-Produktes geschlossen werden. Schließlich eignet sich diese Methode nach einer Kalibrierung zur Konzentrationsbestimmung des Templates, d.h. der Ausgangskonzentration der zu amplifizierenden Nukleinsäure.

In WO 97/29210 und US 5,716,784 ist ein sog. Taqman-Verfahren beschrieben, das eine in situ- und sequenzspezifische Detektion von PCR-Produkten ermöglicht. Bei dem Taqman-Verfahren werden Primer eingesetzt, die zwei Fluoreszenzfarbstoffe in unmittelbarer Nähe zueinander tragen. Dadurch überträgt der angeregte Farbstoff durch Resonanztransfer seine 'Energie' auf den zweiten Farbstoff, der rotverschoben das Fluoreszenzlicht aussendet. Wird der Primer durch die Taq-Polymerase eingebaut, so verliert er durch die Endonukleaseaktivität der Polymerase den zweiten Farbstoff, so dass das ausgesendete Fluoreszenzlicht blauverschoben ist. Durch diese Fluoreszenzlichtverschiebung wird das PCR-Produkt detektiert. Nach einer Kalibrierung eignet sich diese Methode ebenfalls zur Konzentrationsbestimmung des Templates.

Ferner sind von der Fa. Roche Diagnostics GmbH, Roche Molecular Biochemicals, Mannheim sog. Hybridization Probes beschrieben. Derartige Hybridization Probes sind hybridisierungsfähige Oligonukleotide, die an ihren Enden zwei unterschiedliche Fluoreszenzfarbstoffe tragen, die in Fluoreszenzenergie-Resonanztransfer (FRET) treten können. Die Hybridization Probes werden so gewählt, dass sie auf dem PCR-Produkt nebeneinander hybridisieren, und dadurch eine Rotverschiebung des Fluoreszenzlichtes durch den Energieresonanztransfer der beiden nebeneinanderliegenden Farbstoffe einsetzt. Diese Farbverschiebung wird gemessen, um das PCR-Produkt zu detektieren. Ein wesentlicher Nachteil der Methode ist, dass die Konsensussequenz des PCR-Produktes bekannt sein muss. Diese Methode eignet sich ebenfalls nach einer Kalibrierung zur Konzentrationsbestimmung des Templates.

Nachteilig an den vorstehend beschriebenen Verfahren ist, dass für den Nachweis der in einer Polymerase-Kettenreaktion amplifizierten Nukleinsäuremoleküle eine Markierung der PCR-Produkte erforderlich ist. Ein weiterer Nachteil der Verfahren des Stands der Technik ist, dass üblicherweise zur Bestimmung des Molekulargewichts der PCR-Produkte eine zeitaufwendige elektrophoretische Aufarbeitung der PCR-Produkte erforderlich ist.

Es besteht daher ein Bedarf an alternativen Nachweisverfahren für amplifizierte Nukleinsäuremoleküle, bei denen auf eine Markierung der PCR-Produkte verzichtet werden kann, und die gleichzeitig eine Detektion der PCR-Produkte in situ während der PCR ermöglichen.

Aufgabe der vorliegenden Erfindung ist es somit, ein Verfahren bereitzustellen, mit dem in einer Polymerase-Kettenreaktion amplifizierte Nukleinsäuremoleküle qualitativ und/oder quantitativ nachgewiesen werden können, ohne dass eine Markierung der PCR-Produkte beispielsweise mit Färbemethoden oder eine elektrophoretische Aufarbeitung der PCR-Produkte erforderlich ist.

Eine weitere Aufgabe der vorliegenden Erfindung besteht darin, ein Verfahren bereitzustellen, mit dem das Molekulargewicht von in einer Polymerase-Kettenreaktion amplifizierten Nukleinsäuremolekülen bestimmt werden kann, ohne dass eine elektrophoretische Aufarbeitung der PCR-Produkte erforderlich ist.

Ferner ist es eine Aufgabe der vorliegenden Erfindung, dass der Nachweis der PCR-Produkte in situ während der PCR möglich ist, um eine Optimierung der PCR-Dauer auf die für die gewünschte Menge an PCR-Produkt ausreichende Zahl an Amplifikations-Zyklen zu erreichen.

Eine weitere Aufgabe der vorliegenden Erfindung besteht in der Bereitstellung eines Verfahrens, das die Ermittlung der Ausgangskonzentration des Templates ermöglicht.

Weitere Aufgaben ergeben sich aus der nachfolgenden Beschreibung.

Die Aufgaben der vorliegenden Erfindung werden durch die Bereitstellung der in den Patentansprüchen gekennzeichneten Ausführungsformen gelöst.

Überraschenderweise wurde jetzt gefunden, dass in einer Polymerase-Kettenreaktion amplifizierte Nukleinsäuremoleküle qualitativ und/oder quantitativ nachgewiesen werden können, indem die Änderung der Masse an Oligonukleotiden in einer Amplifikationslösung im Verlauf einer Polymerase-Kettenreaktion (PCR) detektiert wird.

Die in einer Amplifikationslösung vorliegenden Oligonukleotide sind die zu amplifizierenden Template-Nukleinsäuremoleküle, die amplifizierten Nukleinsäuremoleküle sowie die eingesetzten Oligonukleotidprimer. Die zu amplifizierenden Template-Nukleinsäuremoleküle und die amplifizierten Nukleinsäuremoleküle werden im Folgenden auch unter dem Begriff Target-DNA zusammengefasst.

Im Folgenden werden einige Begriffe erläutert, die zur Beschreibung der vorliegenden Erfindung verwendet werden.

Unter SMO wird im Rahmen der vorliegenden Erfindung ein Sensorsystem zum Molekulargewichts-spezifischen Nachweis von Oligonukleotiden bezeichnet.

Unter SMO-PCR wird im Rahmen der vorliegenden Erfindung ein Sensorsystem zum Molekulugewichts-spezifischen Nachweis von Oligonukleotiden während der PCR verstanden.

Eine Polymerase-Kettenreaktion (polymerase chain reaction, PCR) umfasst im Rahmen der vorliegenden Erfindung üblicherweise etwa 10 bis 50 oder mehr PCR-Zyklen, vorzugsweise etwa 20 bis 40 PCR-Zyklen, besonders bevorzugt etwa 30 Zyklen.

Als PCR-Zyklus wird im Rahmen der vorliegenden Erfindung ein einzelner Verstärkungsschritt der PCR bezeichnet.

Als PCR-Produkt wird im Rahmen der vorliegenden Erfindung ein Produkt aus der Verstärkung bzw. Vervielfältigung der zu amplifizierenden Nukleinsäuremoleküle durch die PCR bezeichnet.

Als Oligonukleotidprimer bzw. Primer wird im Rahmen der vorliegenden Erfindung ein Oligonukleotid bezeichnet, das an die Target-DNA bindet bzw. hybridisiert, wobei von der Bindungsstelle die Synthese des Gegenstranges der Target-DNA bei der PCR startet.

Unter Target bzw. Target-DNA wird im Rahmen der vorliegenden Erfindung die DNA verstanden, die bei der PCR kopiert wird.

Unter Template bzw. Template-DNA wird im Rahmen der vorliegenden Erfindung die DNA verstanden, die durch die PCR verstärkt bzw. amplifiziert werden soll.

Unter Amplifikationslösung wird im Rahmen der vorliegenden Erfindung ein Reaktionsgemisch verstanden, mit dem die PCR durchgeführt wird.

Die Bezeichnung dNTP steht im Rahmen der vorliegenden Erfindung für Desoxyribonukleotidtriphosphat, das Monomer, das als Edukt für die PCR benötigt wird.

Die Bezeichnungen A, G, C und T stehen für Adenin, Guanin, Cytosin und Thymin.

Als Oligonukleotid wird im Rahmen der vorliegenden Erfindung ein Polymer aus Desoxyribonukleotidtriphosphaten bezeichnet.

Als Probenlösung wird im Rahmen der vorliegenden Erfindung die zu analysierende Flüssigkeit mit den zu amplifizierenden Nukleinsäuremolekülen bezeichnet.

Unter der Denaturierungstemperatur wird im Rahmen der vorliegenden Erfindung die Temperatur verstanden, bei der die doppelsträngige DNA im PCR-Zyklus aufgetrennt wird. Die Denaturierungstemperatur beträgt üblicherweise mehr als 90°C, vorzugsweise etwa 95°C.

Unter der Annealingtemperatur wird im Rahmen der vorliegenden Erfindung die Temperatur verstanden, bei der die Primer an das Target hybridisieren. Die Annealingtemperatur liegt üblicherweise im Bereich von 60°C bis 70°C und beträgt vorzugsweise etwa 65°C.

Unter der Kettenverlängerungs- bzw. Extensionstemperatur wird im Rahmen der vorliegenden Erfindung die Temperatur verstanden, bei der die DNA durch Einbau der Monomerbausteine synthetisiert wird. Die Extensionstemperatur liegt üblicherweise im Bereich von etwa 70°C bis etwa 75°C und beträgt vorzugsweise etwa 72°C.

Unter Hybridisierung wird im Rahmen der vorliegenden Erfindung die Assoziation zweier einzelsträngiger DNA-Fragmente zu einem Doppelstrang verstanden. Dabei findet die Assoziation vorzugsweise immer zu Paaren von A und T bzw. G und C statt.

Unter einer Universaloligonukleotid-Sensorfläche (UvOS-Fläche) wird im Rahmen der vorliegenden Erfindung eine Oberfläche verstanden, auf der sämtliche kombinatorisch erzeugbaren Oligonukleotide mit einer definierten Länge gleich verteilt gebunden sind.

Als Detektionsphase wird im Rahmen der vorliegenden Erfindung die Phase bezeichnet, in der die Bestimmung der Massenänderung der Oligonukleotide während der PCR stattfindet.

Als Amplifikationsphase wird im Rahmen der vorliegenden Erfindung die Phase bezeichnet, in der die PCR stattfindet.

Die Erfindung beruht auf der Tatsache, dass sich bei jedem PCR-Zyklus die Masse, aber nicht die Menge an Oligonukleotiden vergrößert. Dies wird im Folgenden an einem Beispiel ausgehend von der Amplifikation eines einzelsträngigen Produkts erläutert. Zu Beginn einer PCR befinden sich beispielsweise 10 μmol Oligonukleotidprimer und 1 μmol Template-Nukleinsäuremolekül in der Amplifikationslösung. Nach zwei PCR-Zyklen befinden sich bei einer idealen PCR mit einem Verstärkungsfaktor von 2 in der Lösung 4 μmol Produkt und, da Primer zur Amplifikation verbraucht wurden, 7 μmol Primer. Da die Masse der Primer in der Regel nur etwa 1/10 des Produktes beträgt, nimmt die Masse an Oligonukleotiden, umfassend die Masse an Target-DNA und die Masse an Oligonukleotidprimern, nach zwei Zyklen in diesem Beispiel um etwa das 2,4-fache zu. Beträgt die Konzentration an Primern zu Beginn der PCR das 100-fache der Konzentration an Template-Nukleinsäure, würde bei diesem Beispiel die Gesamtmasse an Oligonukleotiden nach zwei PCR-Zyklen um den Faktor 1,03 zunehmen. Die Massenzunahme entsteht jeweils durch den Einbau an Desoxyribonukleotidtriphosphat-Monomeren (dNTP), die zur Synthese des PCR-Produkts dienen.

Gegenstand der vorliegenden Erfindung ist somit ein Verfahren zum qualitativen und/oder quantitativen Nachweis von in einer Polymerase-Kettenreaktion amplifizierten Nukleinsäuremolekülen, bei dem die Änderung der Masse an Oligonukleotiden in einer Amplifikationslösung, umfassend die Masse an Target-DNA und ggf. die Masse an Oligonukleotidprimern, während der Polymerase-Kettenreaktion (PCR) bestimmt wird.

Ein wesentlicher Vorteil des erfindungsgemäßen Verfahrens besteht darin, dass für den Nachweis der PCR-Produkte im Gegensatz zu allen bislang bekannten Verfahren eine Markierung der amplifizierten Nukleinsäuremoleküle beispielsweise durch Färbemethoden nicht erforderlich ist, sondern der Nachweis einer erfolgreichen Amplifikation während der Amplifikationsreaktion allein über die Masse der gebildeten PCR-Produkte erfolgt. Daraus ergibt sich, dass die bei den Nachweisverfahren des Standes der Technik anfallenden Kosten insbesondere für Farbstoffe wie beispielsweise Fluoreszenzfarbstoffe bei dem erfindungsgemäßen Verfahren entfallen. So können beispielsweise die Kosten für die speziellen bei dem Taqman-Verfahren eingesetzten fluoreszenzmarkierten Primer, die zwei Fluoreszenzfarbstoffe in unmittelbarer Nähe zueinander tragen, eingespart werden.

Ein weiterer Vorteil des erfindungsgemäßen Verfahrens ist, dass eine Polymerase-Kettenreaktion, bei der Markierungsmethoden, beispielsweise in Form von Färbemethoden, zum Nachweis der amplifizierten Nukleinsäuremoleküle überflüssig sind, wesentlich robuster, d.h. mit einem geringeren Risiko eines Reaktionsabbruchs und/oder mit einem höheren Verstärkungsfaktor, abläuft.

Es versteht sich für den Fachmann, dass das erfindungsgemäße Nachweisverfahren nicht auf den Einsatz bei PCR-Reaktionen beschränkt ist. Vielmehr kann die Erfindung auch auf andere Amplifikationsreaktionen angewendet werden. Beispielhaft seien hier genannt die sog. IDA-Reaktion (siehe DE 197 41 714), die Ligase-Kettenreaktion (LCR-Methode), die P&LCR, die 3SR-Reaktion (self-sustained sequence replication), die NASBA-Methode (nucleic acid sequence based amplification), die SDA-Reaktion (strand displacement amplification). Es ist klar, dass sich das erfindungsgemäße Nachweisverfahren für alle Reaktionen eignet, während deren Verlauf Nukleinsäuremoleküle amplifiziert werden, also die Änderung der Masse an Nukleinsäuren eine Aussage über den Amplifikationserfolg zulässt.

Vorzugsweise erfolgt die Bestimmung der Änderung der Masse an Oligonukleotiden während der PCR durch Bestimmung der Masse an bei einem PCR-Zyklus vorliegenden Oligonukleotiden bei mindestens zwei PCR-Zyklen. Besonders bevorzugt erfolgt die Bestimmung der Masse an bei einem PCR-Zyklus vorliegenden Oligonukleotiden bei jedem PCR-Zyklus. Alternativ kann die Masse an Oligonukleotiden in der Amplifikationslösung bzw. PCR-Reaktionslösung aber auch bei jedem zweiten Zyklus oder jedem dritten Zyklus oder in beliebigen anderen Intervallen bestimmt werden. Um eine Änderung der Masse an Oligonukleotiden während der PCR zu bestimmen, ist es allerdings erforderlich, dass die Masse an Oligonukleotiden bei mindestens zwei Zyklen der PCR bestimmt wird.

Bei einer bevorzugten Ausführungsform des erfindungsgemäßen Verfahrens wird spezifisch die Zunahme der Masse an amplifizierten Nukleinsäuremolekülen ohne Berücksichtigung der Masse an Oligonukleotidprimern während der PCR bestimmt. Alternativ ist es jedoch ebenso vorteilhaft, die Änderung der Gesamtmasse an Oligonukleotiden, d.h. die Masse an DNA, die bei der PCR kopiert wird und die Masse an Oligonukleotidprimern, während der PCR zu bestimmen.

Vorzugsweise erfolgt die Bestimmung der Masse an bei einem PCR-Zyklus vorliegenden Oligonukleotiden durch Bestimmung der Masse an Oligonukleotiden, die an eine Oberfläche binden. Besonders bevorzugt wird bei dem erfindungsgemäßen Verfahren eine Oberfläche verwendet, die PCR-Produkt und Primer mit in etwa gleicher Affinität bindet. Es kann allerdings ebenfalls bevorzugt sein, dass eine Oberfläche verwendet wird, die bevorzugt die Target-DNA bindet. Bei beiden Alternativen ist es vorteilhaft, wenn die bei der PCR als MonomerBausteine eingesetzten Desoxyribonukleotidtriphosphate nicht an die Oberfläche binden. Ferner ist es bevorzugt, dass die Bindung der Oligonukleotide an die Oberfläche reversibel erfolgt.

Bei einer besonders bevorzugten Ausführungsform des erfindungsgemäßen Verfahrens erfolgt die Bestimmung der Änderung der Masse an Oligonukleotiden in situ, d.h. in der Reaktionskammer, in der auch die PCR abläuft. Auf diese Weise kann die für eine gewünschte Menge an Target-DNA erforderliche Anzahl an PCR-Zyklen online bestimmt werden. Wird beispielsweise die Bildung der gewünschten Menge an Target-DNA detektiert, kann die PCR automatisch abgebrochen werden. Somit kann die Zyklenzahl bei der PCR durch das erfindungsgemäße Verfahren auf die für die Menge an Target-DNA ausreichende Anzahl an Zyklen beschränkt werden. Diese in situ-Optimierung der Reaktionsdauer erlaubt eine Zeit- und Kostenersparnis.

Bei einer weiteren bevorzugten Ausführungsform werden nach jedem Amplifikationsschritt auf der Oberfläche vorliegende Bindungsstellen mit einem Oligonukleotid abgesättigt. Da die Masse bei gleicher Molzahl an Oligonukleotiden mit jedem PCR-Zyklus zunimmt, nimmt auch die an der Oberfläche gebundene Masse mit jedem PCR-Zyklus zu.

Die Bestimmung der Änderung der Masse an Oligonukleotiden während der PCR erfolgt bei dem erfindungsgemäßen Verfahren vorzugsweise durch einen massensensitiven Detektor. Als massensensitiver Detektor kann beispielsweise ein Transducer verwendet werden, der eine Massenänderung auf einer Oberfläche in ein elektrisches oder optisches Signal wandelt. Als Detektionsverfahren können allgemein im Rahmen der vorliegenden Erfindung solche Verfahren eingesetzt werden, die sensitiv auf eine Massenänderung auf einem Oberflächenfilm reagieren. Es ist allerdings nicht erforderlich, dass die Messgröße die Massenänderung selbst ist. So können auch alle von der Masse abhängigen Messgrößen wie z.B. Brechungsindex, Dichteänderung, Schichtdickenänderung und Kompressibilität sowie deren Kombinationen zur Bestimmung der Änderung der Masse an Oligonukleotiden bei dem vorliegenden Verfahren herangezogen werden.

Folgende Detektoren bzw. Transducer werden besonders bevorzugt bei dem erfindungsgemäßen Verfahren eingesetzt:

- Oberflächenwellensensoren bzw. Surface Acoustic Wave (SAW)-Sensoren, bei denen rückgekoppelt die Eigenschwingung der Sensorfläche gemessen wird, die in starkem Maße von ihrer Masse abhängt (N. Barie, M. Rapp, H. J. Ache, UV crosslinked polysiloxanes as new coating materials for SAW devices with high long-term stability, Sensors and Actuators B, 46(1998) S.97-103).

- Mikrowaagen auf der Basis eines Schwingquarzes, der seine Eigenfrequenz mit der Oberflächenbeladung ändert (G. Schwedt; Analytische Chemie: Grundlagen, Methoden und Praxis; Stuttgart [u.a.] : Thieme, 1995).

- Sensoren basierend auf Quartz Crystal Microbalance Dissipation, einem auf der Basis von Mikrowaagen erweiterten Verfahren, welches die Dämpfung der Quarzschwingung zusätzlich ausnützt (Fa. Q-Sense, Göteborg, Schweden).

- Detektoren basierend auf interferometrischen Verfahren, bei denen Brechungsindex und Schichtdickenänderungen gemessen werden.
Ein besonders bevorzugtes interferometrisches Verfahren ist im Rahmen der vorliegenden Erfindung die Weißlichtinterferometrie (W. Nebe, Analytische Interferometrie, Leipzig: Akad. Verl.-Ges., 1970), die im Falle der analytischen Messung von Schichtdickenänderungen auch als RIFS (Reflectometric Interference Spectroscopy) bezeichnet wird (G. Gauglitz, A. Brecht, G. Kraus, W. Nahm; Chemical and biochemical sensors based on interferometry at thin (multi-)layers; Sensors and Actuators B, 11 (1993) 21-27).

- Detektoren zur Bestimmung von Oberflächenplasmonenresonanz.
Bei der Oberflächenplasmonenresonanz-Spektroskopie (Surface Plasmonen Resonance Spectroscopy, SPR) wird die Winkelabhängigkeit der Reflexionsintensität z.B. an dem Grenzflächensystem Glas-Gold-Sensorfläche-Amplifikationslösung gemessen (I. Stemmler, A. Brecht, G. Gauglitz; Compact SPR-transducers with spectral readout for biosensing applications; Sens. Actuators B 54, 98 -105 (1999)). Die Plasmonenresonanzfrequenz hängt in hohem Maße von der Dichte der Sensorfläche ab. Mit der Änderung der Plasmonenresonanzfrequenz ändert sich auch die Reflexionsintensität.

Bei einer weiteren bevorzugten Ausführungsform umfasst das erfindungsgemäße Verfahren die folgenden Schritte:

a) Denaturierung von DNA-Doppelsträngen durch Einstellung einer geeigneten Temperatur (Denaturierungstemperatur);
b) Annealing der Oligonukleotidprimer an die zu amplifizierenden Nukleinsäuremoleküle durch Einstellung einer geeigneten Temperatur (Annealingtemperatur);
c) DNA-Synthese bzw. Kettenverlängerung bei einer geeigneten Temperatur (Kettenverlängerungstemperatur);
d) ggf. Wiederholung der Schritte a) bis c),

wobei die Bestimmung der Masse an bei einem PCR-Zyklus vorliegenden Oligonukleotiden nach Schritt b) und/oder nach Schritt c) erfolgen kann.

Bei der PCR werden für jeden PCR-Zyklus herkömmlicherweise drei Temperaturen durchfahren. Vorzugsweise lösen sich die an der Oberfläche gebundenen Oligonukleotide bei der höchsten Temperatur, d.h. der Denaturierungstemperatur von der Oberfläche ab. Ein bevorzugter Wert für die Denaturierungstemperatur beträgt 95°C. Somit kann bei dieser Denaturierungstemperatur ein Messwert bestimmt werden, der als Nullwert bzw. Bezugswert für die bei dem jeweiligen PCR-Zyklus bestimmte Masse an Oligonukleotiden dient.

Bei der im PCR-Zyklus nachfolgenden Temperatur, der Annealingtemperatur von beispielsweise etwa 65°C, werden die Oligonukleotide wieder vollständig von der Oberfläche, beispielsweise der Sensorfläche, adsorbiert. Bei einer Ausführungsform des erfindungsgemäßen Verfahrens erfolgt deswegen die Bestimmung der Masse an bei einem PCR-Zyklus vorliegenden Oligonukleotiden bei der Annealing-Temperatur.

Um die Empfindlichkeit des erfindungsgemäßen Verfahrens zu erhöhen, kann es ferner vorteilhaft sein, die Temperatur unter die Annealingtemperatur zu senken, so dass die Bestimmung der Masse an bei einem PCR-Zyklus vorliegenden Oligonukleotiden bevorzugt bei einer Temperatur unterhalb der Annealing-Temperatur erfolgt. Beispielsweise kann die Massenbestimmung bei einer Temperatur im Bereich von 25°C bis 50°C und vorzugsweise im Bereich von 30°C bis 40°C erfolgen.

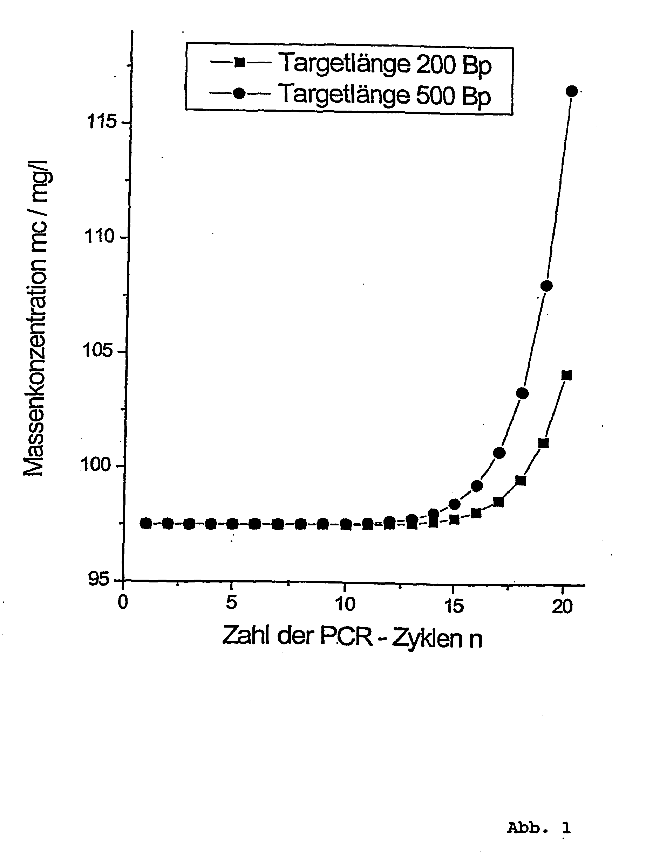

Wird von dem bei der bzw. unterhalb der Annealing-Temperatur erhaltenen Messwert der bei der Denaturierungstemperatur bestimmte Nullwert bzw. Bezugswert abgezogen, so erhält man ein von Störeinflüssen freies Messergebnis, in dem Schwankungen und Drift eliminiert sind. Dieses Ergebnis ist bei Verwendung von geeigneten massensensitiven Messverfahren proportional zur Masse $mc_n$ an Oligonukleotiden nach n Zyklen. Das Ergebnis eines derartigen Nachweisverfahrens ist in Abbildung 1 dargestellt.

Bei einer besonders bevorzugten Ausführungsform des erfindungsgemäßen Verfahrens wird aus der Änderung der Masse an Oligonukleotiden während der PCR das Molekulargewicht der amplifizierten Nukleinsäuremoleküle und/ oder der Verstärkungsfaktor der Polymerase-Kettenreaktion bestimmt.

Die Bestimmung des Molekulargewichts der amplifizierten Nukleinsäuremoleküle und/oder des Verstärkungsfaktors der PCR erfolgt über die im Folgenden angegebenen Gleichungen. Dabei steht M für das Messergebnis, das sich beispielsweise bei der Detektion der Massenänderung durch WeißlichtInterferometrie aus dem Produkt von Brechungsindexdifferenz $\Delta n$ und Schichtdickenänderung $\Delta d$ ergibt. $cp_n$ steht für die Primerkonzentration nach n Zyklen, während $ct_n$ für die Targetkonzentration nach n Zyklen steht. n gibt die Zyklenanzahl der PCR an und v bezeichnet den Verstärkungsfaktor der PCR. $mc_n$ gibt die Masse an Oligonukleotiden nach n Zyklen an, lp und lt stehen für die Länge der Primer bzw. die Länge des Targets und PCR-Produktes der PCR in Anzahl der Basen, während MB für das mittlere Molgewicht der vier Basen steht, das mit 325 g/Mol angesetzt werden kann. Alle folgenden Ableitungen und Konzentrationen beziehen sich auf einzelsträngige DNA.

Die Targetkonzentration nach 0 Schritten beträgt: $ct_0 = ct_0 * v^0$

Bei einer idealen PCR beträgt der Vervielfältigungsfaktor v = 2. Es findet dann mit jedem Schritt, d.h. jedem PCR-Zyklus eine Verdopplung der Targetkonzentration statt:

Nach dem 1. Schritt beträgt die Targetkonzentration $ct_1 = ct_0 * v$, nach dem 2. Schritt $ct_2 = ct_1 * v = ct_0 v^2$ und nach dem n-ten Schritt $ct_n = ct_0 * v^n$.

Bei jedem Schritt, bei dem Target gebildet wird, wird Primer verbraucht und zwar mit jedem Targeteinzelstrang ein Primereinzelstrang. Daher beträgt die Primerkonzentration nach dem 1. Schritt: $cp_1 = cp_0 - ct_0$, nach dem zweiten Schritt: $cp_2 = cp_1 - ct_1 = cp_0 - ct_0 - ct_1 = cp_0 - ct_0 - ct_0 v$, nach dem dritten Schritt: $cp_3 = cp_2 - ct_2 = cp_0 - cto * (1 - v - v^2)$ und nach dem n-ten Schritt: $cp_n = cp_0 - ct_0 * (1 - v - v^2 - v^3 .... v^n)$

Der Klammerausdruck stellt eine geometrische Folge dar. Daher wird $cp_n$ zu:

$$cp_n = cp_0 - ( ct_0 * (v^n - 1) / (v - 1) )$$

Die Targetlänge lt, die nach Mulitplikation mit dem mittleren Molekulargewicht der Basen das Molekulargewicht der Target-DNA ergibt, und der Verstärkungsfaktor v lassen sich auf folgende Weise ermitteln:

Die Massenkonzentration $mc_n$ an Oligonukleotiden ergibt sich, indem die Konzentrationen an Primer mit der Primerlänge lp und an Target mit der Targetlänge lt multipliziert werden und das Ergebnis mit dem mittleren Molgewicht der Basen MB multipliziert wird:

$$mc_n = MB * cp_n * lp + MB * ct_n * lt$$

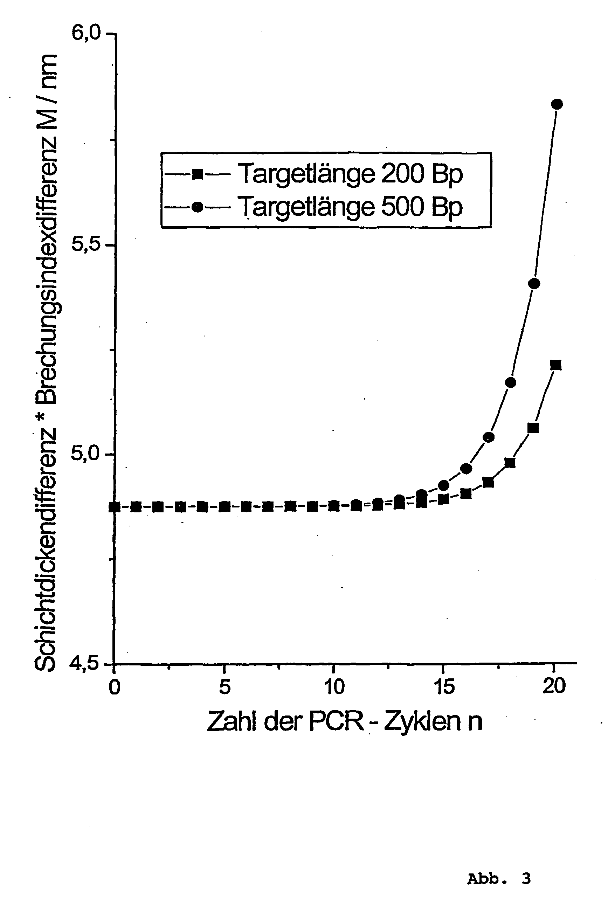

Hierbei macht man die Annahme, dass für die ersten PCR- Schritte (in Abbildung 3 bis etwa Schritt 20) MB * $cp_n$ * lp nahezu konstant bleibt, da der Primerverbrauch zur Gesamtmenge an Primer in der Lösung während der ersten PCR-Schritte bleibt.

Deshalb kann eine Konstante K = MB* $cp_n$ * lp eingeführt werden.

Das Meßsignal $M_n$ hängt von der Gesamtmassenkonzentration über die Systemkonstante E linear ab: $M_n = E * mc_n$

Während der ersten Schritte wird das Signal nur durch die Primer hervorgerufen, da im Vergleich zur Primerkonzentration so gut wie kein Target erzeugt wurde: $M_{start}$ entspricht dann $E * MB * cp_n * lp = K * E$

Das gesamte Messsignal ergibt sich aus der Summe von $M_{start}$ und $M'_n$. $M'_n = E * MB * ct_n * lt = E * MB * ct_0 * v^n * lt$.

Da nur v und lt von Interesse sind, wird $M_{start}$ von $M_n$ abgezogen :

$$M'_n = M_n - M_{start}$$

Da als zweiter Messwert neben einem Maß für die Masse an Oligonukleotiden nur die Zyklenanzahl bzw. Schrittanzahl n vorliegt und zwischen $M'_n$ und n, bevor eine Sättigungsphase eintritt, ein exponentieller Zusammenhang besteht, werden zur Ermittlung von lt und v die Messwerte $M'_n$ logarithmiert, um anschließend eine lineare Regression durchführen zu können:

$$\log(M'_n) = a * n + b = \log(v) * n + \log(E * MB * lt * ct_0)$$

Durch lineare Regression erhält man a und b und dann durch Umformung die gesuchten Werte Targetlänge lt und Verstärkungsfaktor v.

Die Änderung der Massenkonzentrationen in Abhängigkeit der PCR-Zyklenzahl für ein 500 Basenpaar- und ein 200 Basenpaar-langes Template sind in Abbildung 1 dargestellt.

Der Vorteil der Ermittlung des Molekulargewichts der PCR-Produkte auf die vorstehend beschriebene Weise besteht darin, dass zur Molekulargewichtsbestimmung keine zeitaufwendige elektrophoretische Aufarbeitung der PCR-Produkte im Anschluss an die PCR erforderlich ist.

Bei einer weiteren bevorzugten Ausführungsform des erfindungsgemäßen Vefahrens wird zur Bestimmung der Ausgangskonzentration der Template-DNA eine Reihe von PCR's mit unterschiedlichen und bekannten Ausgangskonzentrationen derselben Nukleinsäuremoleküle, d.h. derselben Templatemoleküle, zur Kalibrierung durchgeführt. Vorzugsweise wird dabei der PCR-Zyklus, bei dem die durch die Amplifikation bewirkte Änderung der Masse an Oligonukleotiden messbar wird, als Maß für die Ausgangskonzentration der Template-Moleküle verwendet. Da die Änderung der Masse an Oligonukleotiden im Verlauf der PCR-Reaktion eine sigmoide Funktion, umfassend einen exponentiellen Anstieg und eine Sättigungsphase, darstellt, kann dieser Startzyklus $n_s$ beispielsweise derart ermittelt werden, dass der Wendepunkt der sigmoiden Funktion bestimmt wird und eine Tangente an die Funktion durch diesen Wendepunkt gelegt wird. Der Schnittpunkt dieser Tangente mit x-Achse (siehe Abb. 1) ergibt den Startzyklus $n_s$. Der Wendepunkt wird durch Bestimmung des Maximums der ersten Ableitung der sigmoiden Funktion erhalten.

Alternativ kann der Startzyklus $n_s$ auch ermittelt werden, indem die Scheitelpunkte der sigmoiden Funktion, d.h. die Maxima der zweiten Ableitung der sigmoiden Funktion, bestimmt werden und durch eine Gerade miteinander verbunden werden. Wiederum ergibt der Schnittpunkt dieser Geraden mit der x-Achse den Startzyklus $n_s$.

Folglich kann der auf die vorstehend beschriebene Weise ermittelte Startzyklus $n_s$ eine reelle Zahl sein.

Auf dieselbe Weise wird der Startzyklus $n_s$ für die Probe mit unbekannter Konzentration ermittelt. Durch Regressions- und Interpolationsrechnung erhält man somit die Startkonzentration $ct_0$ der Template-DNA.

Bei einer weiteren bevorzugten Ausführungsform der vorliegenden Erfindung wird nach Beendigung der PCR die Temperatur bestimmt, bei der sich die Oligonukleotide von der Oberfläche ablösen. Auf diese Weise können Aussagen über die spezifische Natur der PCR-Produkte, insbesondere den GC-Gehalt der amplifizierten Nukleinsäuremoleküle, erhalten werden. Beispielsweise kann im Anschluss an die PCR eine sukzessive Erhöhung der Temperatur durchgeführt werden und dabei die Abschmelztemperatur der Oligonukleotide von Oligonukleotidsonden auf der Oberfläche gemessen werden.

Bei einem weiteren Aspekt der vorliegenden Erfindung wird eine Vorrichtung zum qualitativen und/oder quantitativen Nachweis von in einer Polymerase-Kettenreaktion amplifizierten Nukleinsäuremolekülen bereitgestellt.

Die erfindungsgemäße Vorrichtung umfasst eine Reaktionskammer, in der die PCR durchgeführt wird; eine Einheit zur Steuerung der Temperatur; eine Oberfläche, die zur Bindung von Oligonukleotiden geeignet ist; sowie einen Detektor zur Bestimmung der Masse an auf der Oberfläche gebundenen Oligonukleotiden.

Unter Bindung an eine Oberfläche wird im Rahmen der vorliegenden Erfindung jede mögliche Art von Wechselwirkung der Oligonukleotide mit einer Oberfläche verstanden, die dazu geeignet ist, die Bindung auf der Oberfläche massensensitiv zu bestimmen.

Vorzugsweise befindet sich die Oberfläche auf dem Detektor zur Bestimmung der Masse der auf der Oberfläche gebundenen Oligonukleotide. In dieser Ausführungsform wird die Oberfläche im Rahmen der vorliegenden Erfindung auch als Sensorfläche bezeichnet.

Als Oberfläche bzw. Sensorfläche eignet sich grundsätzlich nahezu jeder Oberflächentyp. Wie in V. Chan et al., Effect of Hydrophobicity and Electrostatics on Adsorption and Surface Diffusion of DNA Oligonucleotides at Liquid/ Solid Interfaces, Journal of Colloid and Interface Science 203, 197 - 207 (1998), beschrieben, binden bzw. adsorbieren Oligonukleotide an fast jede Oberfläche unspezifisch.

Bei einer bevorzugten Ausführungsform bindet die Oberfläche Oligonukleotide unspezifisch, d.h, unabhängig davon, ob es sich um Oligonukleotidprimer oder um üblicherweise wesentlich längere Template-DNA bzw. Target-DNA handelt. Ferner ist es bevorzugt, dass die Oberfläche die Oligonukleotidprimer und Template-DNA bzw. Target-DNA mit ähnlicher Affinität bindet.

Bei einer alternativen Ausführungsform der vorliegenden Erfindung ist die Bindung von amplifizierten Nukleinsäuremolekülen gegenüber der Bindung von Oligonukleotidprimern auf der Oberfläche bevorzugt. In dieser Ausführungsform wird pro PCR-Zyklus eine größere Änderung in der Masse an Oligonukleotiden beobachtet, da der Einfluss der Primerkonzentration geringer ist, bzw., falls die Oligonukleotidprimer nicht an die Oberfläche binden, die Masse der Oligonukleotidprimer nicht detektiert wird.

Ferner ist es bevorzugt, dass die Oberfläche keine Desoxyribonukleotidtriphosphate bindet.

Ferner sind bei einer bevorzugten Ausführungsform der erfindungsgemäßen Vorrichtung Bindungsstellen für die Oligonukleotide auf der Oberfläche gleichmäßig verteilt. Auf diese Weise wird gewährleistet, dass alle Oligonukleotide mit gleicher Wahrscheinlichkeit an die Oberfläche binden.

Bei einer weiteren bevorzugten Ausführungsform der erfindungsgemäßen Vorrichtung ist die Anzahl an Bindungsstellen für die Oligonukleotide auf der Oberfläche größer als die Anzahl an Oligonukleotiden. Dies hat den Vorteil, dass alle Oligonukleotide auf der Oberfläche binden können und somit vergleichbare Massenwerte pro PCR-Zyklus bestimmt werden können.

Als besonders vorteilhafte Ausführungsform der erfindungsgemäßen Vorrichtung hat sich eine Oberfläche erwiesen, bei der die Bindungsstellen auf der Oberfläche angebrachte Oligonukleotidsonden sind, die zur Hybridisierung mit den zu amplifizierenden Nukleinsäuremolekülen, d.h. den Target- und Template-Nukleotidsequenzen, und/oder den Oligonukleotid-Primern geeignet sind.

Dabei ist es besonders bevorzugt, dass alle Oligonukleotidsonden dieselbe Länge aufweisen.

Des Weiteren ist es bevorzugt, dass die Oligonukleotidsonden kombinatorisch auf der Oberfläche erzeugt werden. Eine Oberfläche bzw. Sensorfläche, auf der sämtliche kombinatorisch erzeugbaren Oligonukleotide einer festen Länge gleich verteilt gebunden sind, wird im Folgenden als Universaloligo-Sensorfläche (UvOS)-Fläche bezeichnet. Zur Herstellung einer derartigen UvOS-Fläche wird beispielsweise im Fall eines Glaschips als Träger für die Sensorfläche die Glasoberfläche mit 3-Glycidoxypropyltrimetoxysilan (GOPS) silanisiert (siehe U. Maskos, E.M. Southern, Nucleic Acid Research 1992, 20, 1679), das Epoxid geöffnet und mit Pentaethylenglykol verestert. Anschließend werden mit Hilfe der Phosphoramiditchemie (M.J. Gait, Oligonucleotide Synthesis: A practial Approach; IRL Press at Oxford University Press,1990) oberflächengebundene Oligonukleotide auf die Oberfläche synthetisiert. Dabei werden mit jedem Syntheseschritt alle vier Basen (A, G, C und T) gleichzeitig gekoppelt. Auf diese Weise erhält man nach dem zweiten Schritt alle möglichen 2-mere, d.h. 16 verschiedene Oligonukleotide, gleich verteilt auf der Oberfäche. Nach dem dritten Schritt erhält man alle möglichen 3-mere ($4^3$) und nach dem n-ten Schritt erhält man alle möglichen n-mere ($4^n$) auf der Oberfläche bzw. Sensorfläche. Auf diese Weise wird für jedes denkbare Oligonukleotid eine hybridisierungsfähige Sonde auf der UvOS-Fläche erzeugt. Ein derartiges Verfahren wird als kombinatorische Synthese bezeichnet.

An eine derartige UvOS-Fläche kann jedes sich in der Amplifikationslösung befindende Oligonukleotid binden bzw. hybridisieren. Eine UvOS-Fläche als Sensorfläche erfüllt damit alle Anforderungen, die an einen qualitativen und/ oder quantitativen Nachweis von in einer PCR amplifizierten Nukleinsäuremolekülen durch Bestimmung der Masse der auf der Oberfläche gebundenen Oligonukleotide gestellt werden.

Bei derartigen UvOS-Flächen ist die Hybridisierungstemperatur von wesentlicher Bedeutung. Je tiefer die Temperatur ist, desto besser sind die Bindungen der Oligonukleotide an die Oberfläche. Dagegen wird bei höheren Temperaturen wie z.B. einer Denaturierungstemperatur von etwa 95°C bei der PCR, jegliche DNA-DNA-Wechselwirkung reversibel aufgeschmolzen. Somit lässt sich bei der Denaturierungstemperatur ein Nullwert bzw. Bezugswert für die Bestimmung der Massenänderung bei jedem PCR-Zyklus messen. Für die Bestimmung der Masse der an die Oberfläche gebundenen Oligonukleotide kann es vorteilhaft sein, die Temperatur unter die Annealingtemperatur zu senken, um die Empfindlichkeit des Nachweises zu steigern.

Ferner zeichnen sich bevorzugte Oberflächen bzw. Sensorflächen dadurch aus, dass sie längere DNA-Moleküle besser binden als kürzere. D.h., es werden Template-DNA-Moleküle bzw. Target-DNA-Moleküle besser gebunden als die kürzeren Oligonukleotidprimer. Auf diese Weise verbessert sich das Signal des Nachweises der Amplifizierung von Target-DNA über die Massenänderung.

Um eine Veränderung der Primer- und Target-Konzentration während der Annealingphase durch Bindung an die Oberfläche bzw. Sensorfläche möglichst gering zu halten, ist es des Weiteren bevorzugt, dass das Verhältnis von Sensoroberfläche zu Reaktionskammervolumen möglichst klein gehalten wird. Bevorzugte Werte für das Verhältnis von Oberfläche zu Kammervolumen sind im Bereich von $10^{-1}$ mm$^2$/$\mu$l bis $10^{-3}$ mm$^2$/$\mu$l, besonders bevorzugt 5 x $10^{-2}$ mm$^2$ bis 5 x $10^{-3}$ mm$^2$/$\mu$l und am meisten bevorzugt etwa 0,02 mm$^2$/$\mu$l.

Bevorzugte Detektoren für die Bestimmung der Masse der auf der Oberfläche gebundenen Oligonukleotide wurden bereits vorstehend beschrieben und sind vorzugsweise ausgewählt aus der Gruppe, bestehend aus einem Weißlichtinterferometer, einem Oberflächenwellensensor, einer Mikrowaage, einem Quarzkristall-Microbalance-Dissipation-Sensor sowie einem Sensor zur Detektion von Oberflächenplasmonenresonanz.

Falls beispielsweise die Bestimmung der Masse mittels eines Weißlichtinterferometers erfolgt, ist es vorteilhaft, wenn die Oberfläche der erfindungsgemäßen Vorrichtung eine für die Interferometrie geeignete Dicke vorzugsweise im Bereich von 300 nm bis 700 nm, besonders bevorzugt im Bereich 400 nm bis 600 nm und am meisten bevorzugt von etwa 500 nm aufweist.

Bei einer weiteren bevorzugten Ausführungsform umfasst die Vorrichtung zusätzlich einen Steuerrechner, der die Messdaten aufzeichnet und/oder die Temperatursteuerung sowie den Detektor kontrolliert.

Ferner ist es bevorzugt, dass die erfindungsgemäße Vorrichtung zusätzlich einen Einlass bzw. Auslass für die zu amplifizierenden Proben bzw. amplifizierten Proben umfasst.

Ein weiterer Aspekt der vorliegenden Erfindung ist die Verwendung der vorstehend beschriebenen erfindungsgemäßen Vorrichtung zur Durchführung des erfindungsgemäßen Verfahrens.

Durch die vorliegende Erfindung wird somit ein Sensorsystem zum Molekulargewichts-spezifischen Nachweis von Oligonukleotiden bereitgestellt, das die massenspezifische Detektion von Oligonukleotiden gewährleistet. Besonders vorteilhaft ist der Einsatz der erfindungsgemäßen Vorrichtung zum spezifischen in situ-Nachweis von PCR-Produkten. Auf Grund der Möglichkeit, mit dem vorstehend beschriebenen erfindungsgemäßen Verfahren bzw. mit der erfindungsgemäßen Vorrichtung sowohl die Ausbeute an PCR-Produkten als auch die Basenlänge und damit das Molekulargewicht der PCR-Produkte zu messen, werden übliche zeitaufwendige Analyseschritte wie beispielsweise eine elektrophoretische Aufarbeitung der Produkte, bei der DNA nach ihrer Größe durch Migration in einer Gelmatrix aufgetrennt werden, überflüssig. Durch die Möglichkeit der online-Detektion kann ferner die Zyklenzahl mit Hilfe des erfindungsgemäßen Verfahrens bzw. der erfindungsgemäßen Vorrichtung auf eine für eine gewünschte Menge an PCR-Produkt erforderliche Anzahl an Zyklen beschränkt werden.

Die vorliegende Erfindung wird durch das nachfolgende Ausführungsbeispiel beschrieben, das allerdings nicht als beschränkend ausgelegt werden soll.

Beispiel:

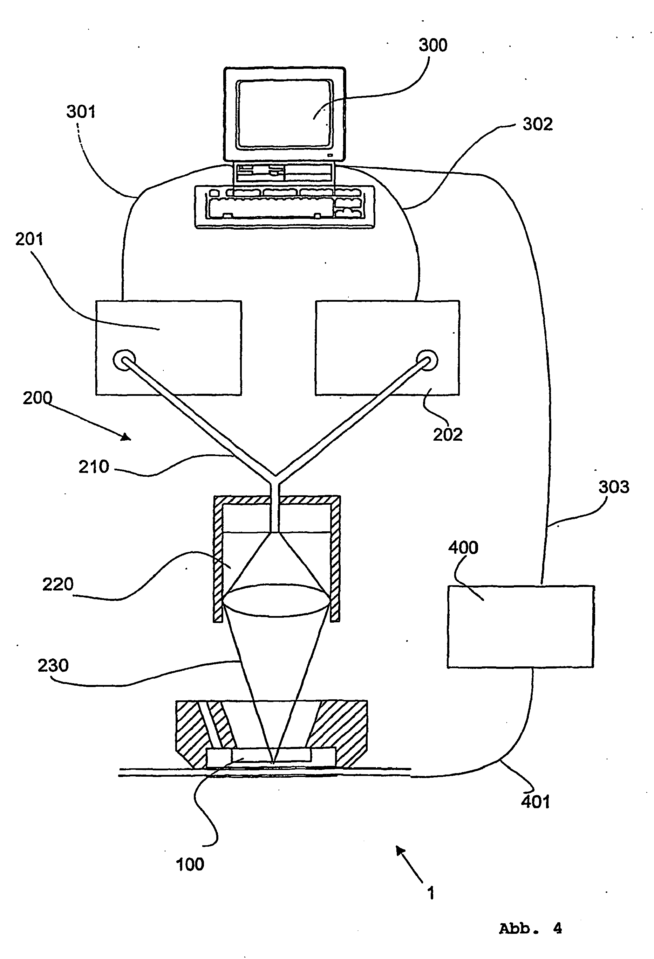

Die erfindungsgemäße Vorrichtung, in diesem Beispiel als SMO-PCR (1) bezeichnet, umfasst in diesem Beispiel ein Weißlicht-Interferometer (200), mit dem die Schichtveränderungen in der Sensoroberfläche (121) der PCR-Sensorzelle (100) gemessen werden (siehe Abbildung 4). Ein Steuerrechner (300) zeichnet die Messdaten über die Steuerleitung (302) auf. Des Weiteren regelt und kontrolliert der Steuerrechner über die Steuerleitungen (301, 303) die Temperatursteuerung (400) sowie die Weißlichtquelle (201).

Aufbau der PCR-Sensorzelle (100)

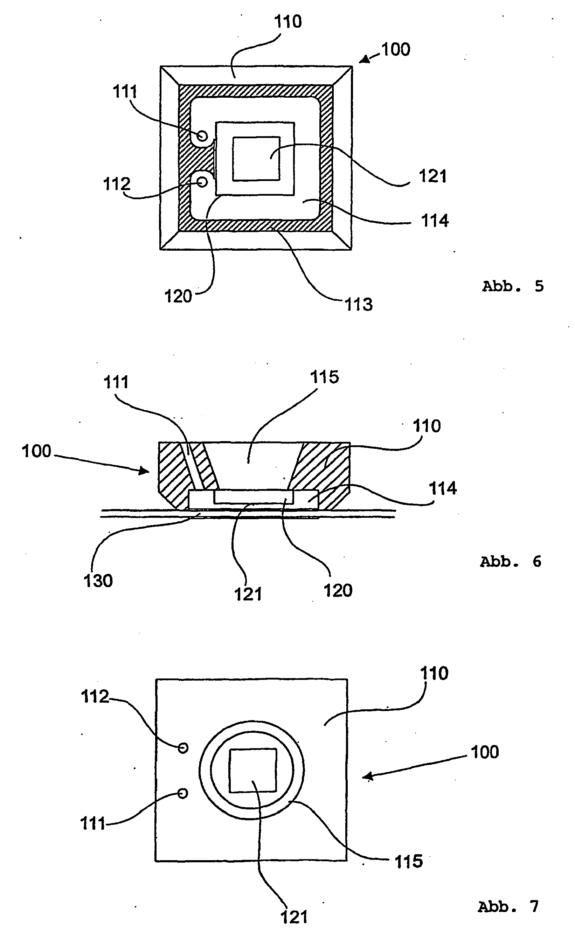

In WO 01/02094 wird eine miniaturisierte PCR-Kammer beschrieben, die durch Einsetzen eines Sensorchips (1) gemäß der vorliegenden Erfindung anstelle des in WO 01/02094 verwendeten Chips im Sinne des erfindungsgemäßen Sensorsystems verwendet werden kann (siehe Abbildung 5). Auf die Offenbarung der WO 01/02094 wird hiermit ausdrücklich Bezug genommen.

Die erfindungsgemäße Vorrichtung bzw. die PCR-Sensorzelle (100) besteht aus einem Gehäuse (110), in der sich eine Kammer (114) befindet, die über einen Auslass (112) und einen Einlass (111) mit Probenlösung entleert und befüllt werden kann. Die Kammer (114) verfügt über einen Lichteintrittskegel (115), um die Wechselwirkungen an der Sensoroberfläche (121) mit einem Weißlicht-Interferometer (200) messen zu können. Der Lichteintrittskegel (115) wird durch den Sensorchip (120) verschlossen. Die Unterseite der Kammer (114) wird mit einer Heizeinheit (130) verklebt und abgedeckt. Mit der Heizeinheit (130) kann die Probenlösung exakt temperiert werden. Eine Heizeinheit ist in WO 01/02094 beschrieben. Das Verhalten der Flüssigkeit sowie die Sicherstellung der Benetzung der Sensorfläche (121) ist ebenfalls in WO 01/02094 beschrieben.

Aufbau des Weißlicht-Interferometers (200)

Das Weißlicht-Interferometer (200), wie es z.B. käuflich bei der Firma ,Ingenieurbüro für Angewandte Spektrometrie', Röntgenstraße 33, D-73431 Aalen zu erhalten ist, besteht aus einer Weißlichtquelle (201), deren Licht über einen Lichtwellenleiter (210) auf eine fokussierende Vorsatzoptik (220) gelangt. Durch die fokussierende Vorsatzoptik (220) wird das Licht auf die Sensorfläche (121) fokussiert. Die von der Sensorfläche (121) reflektierten Lichtstrahlen (230) werden durch die fokussierende Vorsatzoptik (220) in den zweiarmigen Y-Lichtwellenleiter (210) eingekoppelt. Das reflektierte Licht gelangt in das Diodenarray-Spektrometer (202) und wird ausgewertet, indem die Lichtintensität des reflektierten Lichtes in Abhängigkeit von der Wellenlänge gemessen wird.

Sensorchip (120)

Der Sensorchip (120) besteht aus Glas, auf dem ein dreidimensionales $SiO_2$-Netzwerk über Sol-Gel-Technik (C. J. Brinker, G. W. Scherer, Sol-gel science : The physics and chemistry of sol-gel processing; Boston [u.a.] : Academic Press, 1990) aufgebracht wurde, das als Sensorfläche (121) dient. Das Sol-Gel ist etwa 500 nm stark. Es ist mit Glycidoxypropylgruppen modifiziert, an die über Pentaethylenglykol Oligonukleotide synthetisiert werden. Die Oligonukleotidsynthese wird kombinatorisch durchgeführt. Durch diesen Herstellungsweg wird eine mit 500 nm ziemlich dicke und damit für die Weißlicht-Interfemmetrie geeignete UvOS-Fläche erhalten. Der Brechungsindex liegt bei etwa 1,4.

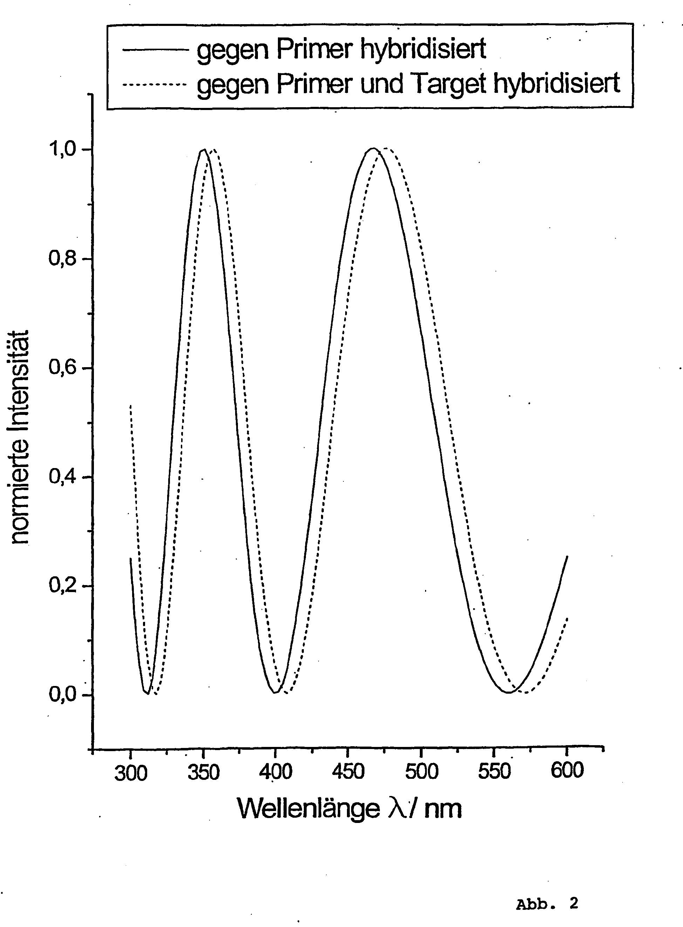

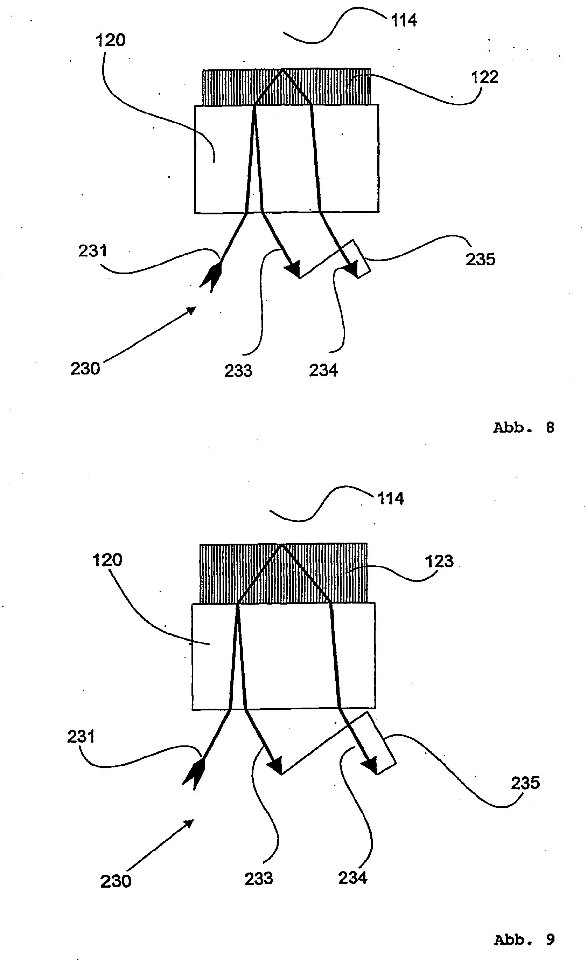

Durch Hybridisierung von Olignukleotiden ändert sich das Produkt aus Brechungsindex und Schichtdicke um etwa 10 nm. Diese Änderung ist schematisch in den Abbildungen 8 und 9 dargestellt. Dort ist auch der Strahlengang des Weißlicht-Interferometers (200) skizziert. Der einfallende Weißlichtstrahl (231) wird an den Grenzflächen Sensorchip (120) und Sensorfläche (121) sowie Sensorfläche (121) und Kammer (114) reflektiert. Die Reflexionen 1. Ordnung (233) und 2. Ordnung (234) zeigen beim Austreten aus dem Sensorchip (120) eine leichte Phasenverschiebung (235). Durch Bindung (Hybridisierung) von Oligonukleotiden wird die Sensorfläche (121) im Allgemeinen dicker. Dadurch wird die Phasendifferenz (235) beim Übergang von der unbeladenen Sensorfläche (122) (siehe Abbildung 8) zur beladenen Sensorfläche (123) (siehe Abbildung 9) größer. Jedoch ist die Änderung der Schichtdicke zu klein, um die Phasendifferenz direkt interferometrisch messen zu können. Daher wird das austretende Licht durch ein Diodenarray-Spektrometer (202) spektral zerlegt und damit die Lichtintensität gegen die Wellenlänge gemessen (siehe Abbildung 2).

Weißlicht-Interferometersignal

Das Interferenzsignal zeigt in Abhängigkeit der Wellenlänge Auslöschungen und Verstärkungen (siehe Abbildung 2). Aus der Verschiebung zweier Spektren lässt sich das Produkt aus Schichtdickenänderung $\Delta d$ und Brechungsindexdifferenz $\Delta n$ der Sensorfläche (121) von einer Messung zur nächsten ermitteln (Peak-zu-Peak-Abstand). Es wird zunächst bei der Denaturierungstemperatur von 95 °C und anschließend bei der Annealingtemperatur von 65°C gemessen. Danach werden die Mittelwerte der Abstände der Minima und Maxima aus den Weißlicht-Interferometerspektren ermittelt. Man erhält damit für jeden PCR-Zyklus als Messwert das Produkt aus Schichtdickenänderung $\Delta d$ und Brechungsindexdifferenz $\Delta n$ zwischen der unbeladenen Sensorfläche bei 95 °C und der beladenen Sensorfläche bei 65 °C. In Abbildung 3 ist ein entsprechendes Beispiel dargestellt.

Datenauswertung (300)

Ein Steuerrechner (300) koordiniert über ein Softwareprogramm die Ansteuerung der Weißlichtquelle (201), der Temperatursteuerung (400) für die PCR und das Auslesen der Messdaten des Diodenarray-Spektrometer (202). Ein Rechenprogramm ermittelt aus den Messdaten des Diodenarray-Spektrometers (202) den Messwert M für jeden PCR-Zyklus (siehe Abbildung 3). Aus der Änderung von M mit jedem PCR-Zyklus lässt sich die Amplifikation pro PCR-Zyklus erkennen. Ein starker Anstieg des Messwertes M weist auf eine erfolgreiche Verstärkung hin. Die Messung kann abgebrochen werden. Aus den erhaltenen Messwertpaaren lässt sich die Länge lt und der Verstärkungsfaktor v der PCR annähernd ermitteln. Dazu wird zunächst der Mittelwert aller Messungen $M_{start}$, bei denen noch keine Amplifikation nachweisbar ist von den übrigen Messungen $M_n$ angezogen:

$$M'_n = M_n - M_{start}$$

Es werden Messwerte $M'_n$ erhalten. Die so erhaltenen Messwertpaare werden logarithmiert und dann durch lineare Regression die Koeffizienten aus der Gleichung

$$\log(M'_n) = a \cdot n + b$$

ermittelt. Daraus lässt sich die PCR-Produktlänge lt und der Verstärkungsfaktor v folgendermaßen berechnen:

$$v = \exp(a),$$

$$lt = \exp(b) / E \cdot ct_0 \cdot MB$$

worin E die Systemkonstante der Messapparatur ist. Für die in Abbildung 3 dargestellten Kurven werden für die Targetlänge lt-Werte von 170 (theor. 200) bzw. 470 bp (theor. 500) erhalten.

Die Startkonzentration des Targets $ct_0$ wird durch eine Kalibrationsmessung erhalten. Es werden mehrere Polymerase-Kettenreaktionen mit demselben Template unterschiedlicher, aber bekannter Startkonzentration vermessen. Für jede PCR wird der Startzyklus $n_s$ ermittelt, ab dem die Amplifikation messbar wird. Da der Startzyklus durch Extrapolationsrechnung erhalten wird, ist $n_s$ eine reelle und keine natürliche Zahl. Genauso wird der Startzyklus von der Probe mit unbekannter Konzentration ermittelt. Durch Regressions- und Interpolationsrechnung erhält man daraus die Startkonzentration $ct_0$.

Abbildungen

Abbildung 1:

Die Masse bzw. Massenkonzentration $mc_n$ an Oligonuleotiden in der Amplifikationslösung wird in Abhängigkeit der Zahl der PCR-Zyklen n für zwei verschiedene Targetlängen (500 Basenpaare und 200 Basenpaare) dargestellt.

Abbildung 2:

Weißlicht-Interferenzsignale werden in Abhängigkeit von der Wellenlänge dargestellt. Die Signale sind auf Werte zwischen 0 und 1 normiert. Deutlich wird die Verschiebung des Signals durch die Änderung der Beladung der Sensorfläche (121) mit dem Target.

Abbildung 3:

Der mit Weißlichtinterferometrie erhaltene Messwert M als Produkt aus Schichtdickendifferenz Δd und Brechungsindexdifferenz An wird in Abhängigkeit von der PCR-Zyklenzahl n für zwei verschiedene Targetlängen (500 Basenpaare und 200 Basenpaare) dargestellt.

Abbildung 4:

Schematische Darstellung der erfindungsgemäßen Vorrichtung in Form eines SMO-PCR-Gesamtaufbaus (1) bestehend aus Steuerrechner (300), Weißlicht-Interferometer (200), PCR-Sensorzelle (100) und Temperatursteuerung (400).

Abbildung 5:

Schematische Darstellung der PCR-Sensorzelle (1) bestehend aus Gehäuse (110) , Einlass (111), Auslass (112), Sensorchip (120) und Reaktionskammer (114).

Abbildung 6:

Schematische Darstellung der PCR-Sensorzelle (1) entlang der Schnittlinie A-A mit angeklebter Heizeinheit (130).

Abbildung 7:

Schematische Darstellung der PCR-Sensorzelle (1) mit Lichteintrittskegel (115) von oben.

Abbildung 8:

Schematische Darstellung des Sensorchips (120) mit unbeladener Sensorfläche (122) und dem Strahlengang bestehend aus einstrahlendem Weißlicht (231) von der Weißlichtquelle (201), der Reflexion 1. Ordnung (233) und der Reflexion 2. Ordnung (234). Anhand der Abbildung wird die wellenlängenabhängige Phasenverschiebung zwischen der Reflexion 1. Ordnung (233) und der Reflexion 2. Ordnung (234) deutlich.

Abbildung 9:

Schematische Darstellung des Sensorchips (120) mit beladener Sensorfläche (123) . Im Vergleich mit Abbildung 8 wird die Änderung der wellenlängenabhängigen Phasenverschiebung in Abhängigkeit der Beladung deutlich. Es sei darauf hingewiesen, dass sich diese Phasenverschiebung nicht direkt messen lässt, da sie mit einer Längenänderung von ca. 10 nm zu klein ist. Daher muss das reflektierte Licht spektral zerlegt werden.

## Patentansprüche

1.  Verfahren zum qualitativen und/oder quantitativen Nachweis auf einer Oberfläche von in einer Amplifikationsreaktion amplifizierten Nukleinsäuremolekülen,
    **dadurch gekennzeichnet, dass** die Oberfläche Nukleinsäuremoleküle aus einer Amplifikationslösung, umfassend Template-Nukleinsäuremoleküle, amplifizierte Nukleinsäuremoleküle und Oligonukleotidprimer, während der in der Amplifikationslösung stattfindenden Amplifikationsreaktion reversibel bindet und die Änderung der Masse der gebundenen Nukleinsäuremoleküle bestimmt wird.

2.  Verfahren nach Anspruch 1,
    **dadurch gekennzeichnet, dass** die Amplifikationsreaktion eine Polymerase-Kettenreaktion (PCR) ist.

3.  Verfahren nach Anspruch 2,
    **dadurch gekennzeichnet, dass** die Bestimmung der Änderung der Masse der Nukleinsäuremoleküle während der PCR durch Bestimmung der Masse der bei einem PCR-Zyklus vorliegenden Nukleinsäuremoleküle bei mindestens zwei PCR-Zyklen, vorzugsweise bei jedem PCR-Zyklus erfolgt.

4.  Verfahren nach einem der vorangehenden Ansprüche,
    **dadurch gekennzeichnet, dass** die Bindung von amplifizierten Nukleinsäuremolekülen gegenüber der Bindung von Oligonukleotidprimern auf der Oberfläche bevorzugt ist.

5.  Verfahren nach einem der Ansprüche 2 bis 4,
    **dadurch gekennzeichnet, dass** bei der PCR als Monomere eingesetzte Desoxyribonukleotidtriphosphate nicht an die Oberfläche binden.

6.  Verfahren nach einem der vorangehenden Ansprüche,
    **dadurch gekennzeichnet, dass** die Bestimmung der Änderung der Masse der Nukleinsäuremoleküle in situ erfolgt.

7.  Verfahren nach einem der vorangehenden Ansprüche,
    **dadurch gekennzeichnet, dass** die Bestimmung der Änderung der Masse der Nukleinsäuremoleküle durch einen massensensitiven Detektor, vorzugsweise ausgewählt aus der Gruppe, bestehend aus einem Weißlichtinterferometer, einem Oberflächenwellensensor, einer Mikrowaage, einem Quartzkristall-Microbalance-Dissipation-Sensor und einem Sensor zur Detektion von Oberflächenplasmonenresonanz, erfolgt.

8.  Verfahren nach einem der Ansprüche 2 bis 7, umfassend die folgenden Schritte:

    a) Denaturierung von DNA-Doppelsträngen durch Einstellung einer geeigneten Temperatur (Denaturierungstemperatur);
    b) Annealing der Oligonukleotidprimer an die zu amplifizierenden Nukleinsäuremoleküle durch Einstellung einer geeigneten Temperatur (Annealingtemperatur);
c) DNA-Synthese bzw. Kettenverlängerung bei einer geeigneten Temperatur (Kettenverlängerungstemperatur);
d) ggf. Wiederholung der Schritte a) bis c),

wobei die Bestimmung der Masse der bei einem PCR-Zyklus vorliegenden Nukleinsäuremoleküle nach Schritt b) und/oder nach Schritt c) erfolgen kann.

9. Verfahren nach einem der vorangehenden Ansprüche,
**dadurch gekennzeichnet, dass** sich die an der Oberfläche gebundenen Nukleinsäuremoleküle bei der Denaturierungstemperatur von der Oberfläche ablösen.

10. Verfahren nach einem der Ansprüche 2 bis 9,
**dadurch gekennzeichnet, dass** bei der Denaturierungstemperatur ein Messwert bestimmt wird, der als Nullwert für die bestimmte Masse der bei einem PCR-Zyklus vorliegenden Nukleinsäuremoleküle dient.

11. Verfahren nach einem der Ansprüche 2 bis 10,
**dadurch gekennzeichnet, dass** die Bestimmung der Masse der bei einem PCR-Zyklus vorliegenden Nukleinsäuremoleküle bei der Annealing-Temperatur erfolgt.

12. Verfahren nach einem der Ansprüche 2 bis 10,
**dadurch gekennzeichnet, dass** die Bestimmung der Masse der bei einem PCR-Zyklus vorliegenden Nukleinsäuremoleküle bei einer Temperatur unterhalb der Annealing-Temperatur, vorzugsweise bei einer Temperatur im Bereich von 25°C bis 50°C und besonders bevorzugt bei einer Temperatur im Bereich von 30°C bis 40°C erfolgt.

13. Verfahren nach einem der Ansprüche 2 bis 12,
**dadurch gekennzeichnet, dass** aus der Änderung der Masse der Nukleinsäuremoleküle während der PCR das Molekulargewicht der amplifizierten Nukleinsäuremoleküle bestimmt wird.

14. Verfahren nach einem der Ansprüche 2 bis 13,
**dadurch gekennzeichnet, dass** aus der Änderung der Masse der Nukleinsäuremoleküle während der PCR der Verstärkungsfaktor der PCR bestimmt wird.

15. Verfahren nach einem der Ansprüche 2 bis 14,
**dadurch gekennzeichnet, dass** zur Bestimmung der Ausgangskonzentration der zu amplifizierenden Nukleinsäuremoleküle vor der PCR eine Reihe von Polymerase-Kettenreaktionen mit unterschiedlichen und bekannten Ausgangskonzentrationen derselben Nukleinsäuremoleküle zur Kalibrierung durchgeführt werden.

16. Verfahren nach Anspruch 15,
**dadurch gekennzeichnet, dass** der PCR-Zyklus, bei dem die durch die Amplifikation bewirkte Änderung der Gesamtmasse an Nukleinsäuremolekülen messbar wird, als Maß für die Ausgangskonzentration der zu amplifizierenden Nukleinsäuremoleküle verwendet wird.

17. Verfahren nach einem der Ansprüche 2 bis 16,
**dadurch gekennzeichnet, dass** nach Beendigung der PCR die Temperatur bestimmt wird, bei der sich die Nukleinsäuremoleküle von der Oberfläche ablösen.

18. Vorrichtung zum qualitativen und/oder quantitativen Nachweis von in einer PCR amplifizierten Nukleinsäuremolekülen, umfassend:

a) eine Reaktionskammer;
b) eine Temperatursteuerungseinheit;
c) eine Oberfläche, auf der alle möglichen n-mere ($4^n$) von Oligonukleotidsonden einer festen Länge n aufgebracht sind; und
d) einen Detektor zur Bestimmung der Masse der auf der Oberfläche gebundenen Nukleinsäuremoleküle.

19. Vorrichtung nach Anspruch 18,
**dadurch gekennzeichnet, dass** die Oberfläche Nukleinsäuremoleküle unspezifisch und/oder mit ähnlicher Affinität bindet.

**20.** Vorrichtung nach Anspruch 18,
**dadurch gekennzeichnet, dass** die Bindung von amplifizierten Nukleinsäuremolekülen gegenüber der Bindung von Oligonukleotidprimern auf der Oberfläche bevorzugt ist.

**21.** Vorrichtung nach einem der Ansprüche 18 bis 20,
**dadurch gekennzeichnet, dass** die Oberfläche keine Desoxyribonukleotidtriphosphate bindet.

**22.** Vorrichtung nach einem der Ansprüche 18 bis 21,
**dadurch gekennzeichnet, dass** Bindungsstellen für die Nukleinsäuremoleküle auf der Oberfläche gleichmäßig verteilt sind.

**23.** Vorrichtung nach Anspruch 18,
**dadurch gekennzeichnet, dass** alle auf der Oberfläche angebrachten Oligonukleotidsonden dieselbe Länge aufweisen.

**24.** Vorrichtung nach einem der Ansprüche 18 bis 23,
**dadurch gekennzeichnet, dass** die Oberfläche silanisiertes Glas umfasst.

**25.** Vorrichtung nach einem der Ansprüche 18 bis 24,
**dadurch gekennzeichnet, dass** die Oberfläche eine für die Interferometrie geeignete Dicke, vorzugsweise im Bereich von 300 nm bis 700 nm, besonders bevorzugt im Bereich 400 nm bis 600 nm und am meisten bevorzugt von etwa 500 nm aufweist.

**26.** Vorrichtung nach einem der Ansprüche 18 bis 25,
**dadurch gekennzeichnet, dass** die Anzahl an Bindungsstellen für die Nukleinsäuremoleküle auf der Oberfläche größer ist als die Anzahl an Nukleinsäuremolekülen.

**27.** Vorrichtung nach einem der Ansprüche 18 bis 26,
**dadurch gekennzeichnet, dass** das Verhältnis von Oberfläche zu Kammervolumen von $10^{-1}$ mm$^2$/$\mu$l bis $10^{-3}$ mm$^2$/$\mu$l, vorzugsweise von 5 x $10^{-2}$/$\mu$l mm$^2$ bis 5 x $10^{-3}$ mm$^2$/$\mu$l und besonders bevorzugt etwa 0,02 mm$^2$/$\mu$l beträgt.

**28.** Vorrichtung nach einem der Ansprüche 18 bis 27,
**dadurch gekennzeichnet, dass** die Vorrichtung zusätzlich einen Steuerrechner umfasst, der die Messdaten aufzeichnet und die Temperatursteuerung sowie den Detektor kontrolliert.

**29.** Vorrichtung nach einem der Ansprüche 18 bis 28,
**dadurch gekennzeichnet, dass** die Vorrichtung zusätzlich einen Probeneinlass und Probenauslass umfasst.

**30.** Vorrichtung nach einem der Ansprüche 18 bis 29,
**dadurch gekennzeichnet, dass** der Detektor ausgewählt ist aus der Gruppe, bestehend aus einem Weißlichtinterferometer, einem Oberflächenwellensensor, einer Mikrowaage, einem Quartzkristall-Microbalance-Dissipation-Sensor und einem Sensor zur Detektion von Oberflächenplasmonenresonanz.

**31.** Verwendung der Vorrichtung nach einem der Ansprüche 18 bis 30 zur Durchführung des Verfahrens nach einem der Ansprüche 1 bis 17.

**Claims**

**1.** A method for qualitative and/or quantitative detection on a surface of nucleic acid molecules amplified in an amplification reaction,
**characterized in that** during the amplification reaction taking place in an amplification solution the surface reversibly binds nucleic acid molecules from the amplification solution, comprising template nucleic acid molecules, amplified nucleic acid molecules and oligonucleotide primers and the change in mass of the bound nucleic acid molecules is determined.

**2.** The method according to claim 1,
**characterized in that** the amplification reaction is a polymerase chain reaction (PCR).

**3.** The method according to claim 2,
**characterized in that** the determination of the change in mass of the nucleic acid molecules during the PCR is performed by determination of the mass of the nucleic acid molecules present at a PCR cycle at at least two PCR cycles, preferably at each PCR cycle.

**4.** The method according to any of the preceding claims,
**characterized in that** the binding of amplified nucleic acid molecules is preferred over the binding of oligonucleotide primers on the surface.

**5.** The method according to any of claims 2 to 4,
**characterized in that** desoxyribonucleotide triphosphates used in the PCR as monomers do not bind to the surface.

**6.** The method according to any of the preceding claims,
**characterized in that** the determination of the change in mass of the nucleic acid molecules is performed in situ.

**7.** The method according to any of the preceding claims,
**characterized in that** the determination of the change in mass of the nucleic acid molecules is performed by a mass-sensitive detector, preferably selected from the group consisting of a white light interferometer, a surface wave sensor, a microbalance, a quartz crystal microbalance dissipation sensor and a sensor for detecting surface plasmon resonance.

**8.** The method according to any of claims 2 to 7, comprising the following steps:

   a) denaturing of DNA double strands by setting a suitable temperature (denaturation temperature);
   b) annealing of the oligonucleotide primers to the nucleic acid molecules to be amplified by setting a suitable temperature (annealing temperature);
   c) DNA synthesis or chain elongation at a suitable temperature (chain elongation temperature);
   d) optionally, repetition of steps a) to c),

wherein the determination of the mass of the nucleic acid molecules present at a PCR cycle can be performed after step b) and/or after step c).

**9.** The method according to any of the preceding claims,
**characterized in that** the nucleic acids bound to the surface detach from the surface at the denaturation temperature.

**10.** The method according to any of claims 2 to 9,
**characterized in that** at the denaturation temperature a value is determined which serves as zero value for the determined mass of the nucleic acid molecules present at a PCR cycle.

**11.** The method according to any of claims 2 to 10,
**characterized in that** the determination of the mass of the nucleic acid molecules present at a PCR cycle is performed at the annealing temperature.

**12.** The method according to any of claims 2 to 10,
**characterized in that** the determination of the mass of the nucleic acid molecules present at a PCR cycle is performed at a temperature below the annealing temperature, preferably at a temperature in the range of from 25°C to 50°C and particularly preferably at a temperature in the range of from 30°C to 40°C.

**13.** The method according to any of claims 2 to 12,
**characterized in that** the molecular weight of the amplified nucleic acid molecules is determined from the change in mass of the nucleic acid molecules during the PCR.

**14.** The method according to any of claims 2 to 13,
**characterized in that** the amplification factor of the PCR is determined from the change in mass of the nucleic acid molecules during the PCR.

**15.** The method according to any of claims 2 to 14,
**characterized in that** for determination of the initial concentration of the nucleic acid molecules to be amplified prior to the PCR a number of polymerase chain reactions with different and known initial concentrations of said nucleic acid molecules are performed for calibration.

16. The method according to claim 15,
**characterized in that** the PCR cycle at which the change of total mass of nucleic acid molecules effected by the amplification becomes measurable is used as a measure for the initial concentration of the nucleic acid molecules to be amplified.

17. The method according to any of claims 2 to 16,
**characterized in that** after completion of the PCR the temperature is determined, at which the nucleic acid molecules detach from the surface.

18. A device for qualitative and/or quantitative detection of nucleic acid molecules amplified by a PCR, comprising:

   a) a reaction chamber;
   b) a temperature control unit;
   c) a surface onto which all possible n-mers ($4^n$) of oligonucleotide probes having a fixed length n are applied; and
   d) a detector for determining the mass of the nucleic acid molecules bound on the surface.

19. The device according to claim 18,
**characterized in that** the surface binds nucleic acid molecules unspecifically and/or with similar affinity.

20. The device according to claim 18,
**characterized in that** the binding of amplified nucleic acid molecules on the surface is preferred over the binding of oligonucleotide primers on the surface.

21. The device according to any of claims 18 to 20,
**characterized in that** the surface does not bind desoxyribonucleotide triphosphates.

22. The device according to any of claims 18 to 21,
**characterized in that** the binding sites for the nucleic acid molecules are evenly distributed on the surface.

23. The device according to claim 18,
**characterized in that** all oligonucleotide probes attached to the surface have the same length.

24. The device according to any of claims 18 to 23,
**characterized in that** surface is silanized glass.

25. The device according to any of claims 18 to 24,
**characterized in that** the surface has a thickness suitable for interferometry, preferably in the range of from 300 nm to 700 nm, particularly preferably in the range of from 400 nm to 600 nm and most preferably of about 500 nm.

26. The device according to any of claims 18 to 25,
**characterized in that** the number of binding sites for the nucleic acid molecules on the surface exceeds the number of nucleic acid molecules.

27. The device according to any of claims 18 to 26,
**characterized in that** the ratio of surface to chamber volume is from $10^{-1}$ mm$^2$/$\mu$l to $10^{-3}$ mm$^2$/$\mu$l, preferably from $5 \times 10^{-2}$ mm$^2$/$\mu$l to $5 \times 10^{-3}$ mm$^2$/$\mu$l and most preferably about 0.02 mm$^2$/$\mu$l.

28. The device according to any of claims 18 to 27,
**characterized in that** the device further comprises a control computer recording the measuring data and controlling the temperature control as well as the detector.

29. The device according to any of claims 18 to 28,
**characterized in that** the device further comprises a sample inlet and a sample outlet.

30. The device according to any of claims 18 to 29,

characterized in that the detector is selected from the group consisting of a white light interferometer, a surface wave sensor, a microbalance, a quartz crystal microbalance dissipation sensor and a sensor for detection of surface plasmon resonance.

**31.** Use of the device according to any of claims 18 to 30 for performance of the method according to any of claims 1 to 17.

**Revendications**

**1.** Procédé de détection qualitative et/ou quantitative sur une surface de molécules d'acide nucléique amplifiées dans une réaction d'amplification,
**caractérisé en ce que** la surface lie de manière résersible des molécules d'acide nucléique à partir d'une solution d'amplification incluant des molécules d'acide nucléique matricielles, des molécules d'acide nucléique amplifiées et des amorces oligonucléotidiques pendant la réaction d'amplification qui se produit dans la solution d'amplification et **en ce que** le changement de masse des molécules d'acide nucléique liées est déterminé.

**2.** Procédé selon la revendication 1,
**caractérisé en ce que** la réaction d'amplification est une réaction en chaîne par polymérase (RCP).

**3.** Procédé selon la revendication 2,
**caractérisé en ce que** la détermination du changement de masse des molécules d'acide nucléique pendant la RCP s'effectue lors d'au moins deux cycles de RCP, préférentiellement lors de chaque cycle de RCP, par détermination de la masse des molécules d'acide nucléique présentes lors d'un cycle de RCP.

**4.** Procédé selon l'une des revendications précédentes,
**caractérisé en ce que** la liaison de molécules d'acide nucléique amplifiées est privilégiée par rapport à la liaison d'amorces oligonucléotidiques sur la surface.

**5.** Procédé selon l'une des revendications 2 à 4,
**caractérisé en ce que** des triphosphates désoxyribonucléotidiques utilisés comme monomères lors de la RCP ne se lient pas à la surface.

**6.** Procédé selon l'une des revendications précédentes,
**caractérisé en ce que** la détermination du changement de masse des molécules d'acide nucléique s'effectue in situ.

**7.** Procédé selon l'une des revendications précédentes,
**caractérisé en ce que** la détermination du changement de masse des molécules d'acide nucléique s'effectue au moyen d'un détecteur sensible à la masse, sélectionné préférentiellement dans le groupe constitué d'un interféromètre en lumière blanche, d'un capteur à ondes de surface, d'une microbalance, d'un capteur de dissipation par microbalance à cristal de quartz et d'un capteur de détection de résonance plasmonique de surface.

**8.** Procédé selon l'une des revendications 2 à 7, comportant les étapes suivantes :

a) dénaturation de doubles brins d'ADN par réglage d'une température adéquate (température de dénaturation) ;
b) hybridation des amorces oligonucléotidiques aux molécules d'acide nucléique à amplifier par réglage d'une température adéquate (température d'hybridation) ;
c) synthèse d'ADN ou allongement de chaîne à une température adéquate (température d'allongement de chaîne) ;
d) éventuellement, répétition des étapes a) à c), la détermination de la masse des molécules d'acide nucléique présentes lors d'un cycle de RCP pouvant s'effectuer après l'étape b) et/ou après l'étape c).

**9.** Procédé selon l'une des revendications précédentes,
**caractérisé en ce que** les molécules d'acide nucléique liées à la surface se détachent de la surface à la température de dénaturation.

**10.** Procédé selon l'une des revendications 2 à 9,
**caractérisé en ce qu'**une valeur de mesure est déterminée à la température de dénaturation pour servir de valeur zéro pour la masse déterminée des molécules d'acide nucléique présentes lors d'un cycle de RCP.

**11.** Procédé selon l'une des revendications 2 à 10,
**caractérisé en ce que** la détermination de la masse des molécules d'acide nucléique présentes lors d'un cycle de RCP s'effectue à la température d'hybridation.

**12.** Procédé selon l'une des revendications 2 à 10,
**caractérisé en ce que** la détermination de la masse des molécules d'acide nucléique présentes lors d'un cycle de RCP s'effectue à une température inférieure à la température d'hybridation, préférentiellement à une température comprise entre 25 °C et 50 °C, et tout préférentiellement à une température comprise entre 30 °C et 40 °C.

**13.** Procédé selon l'une des revendications 2 à 12,
**caractérisé en ce que** le poids moléculaire des molécules d'acide nucléique amplifiées est determiné à partir du changement de masse des molécules d'acide nucléique pendant la RCP.

**14.** Procédé selon l'une des revendications 2 à 13,
**caractérisé en ce que** le facteur de renforcement de la RCP est determiné à partir du changement de masse des molécules d'acide nucléique pendant la RCP.

**15.** Procédé selon l'une des revendications 2 à 14,
**caractérisé en ce que**, pour déterminer la concentration de départ des molécules d'acide nucléique à amplifier avant la RCP, une série de réactions en chaîne par polymérase est effectuée avec des concentrations de départ différentes et connues desdites molécules d'acide nucléique en vue du calibrage.

**16.** Procédé selon la revendication 15,
**caractérisé en ce que** le cycle de RCP lors duquel le changement de la masse totale de molécules d'acide nucléique provoqué par l'amplification est mesurable, est utilisé comme grandeur pour la concentration de départ des molécules d'acide nucléique à amplifier.

**17.** Procédé selon l'une des revendications 2 à 16,
**caractérisé en ce que** la température à laquelle les molécules d'acide nucléique se détachent de la surface est déterminée après achèvement de la RCP.

**18.** Dispositif de détection qualitative et/ou quantitative de molécules d'acide nucléique amplifiées dans une RCP, comportant :

   a) une chambre de réaction ;
   b) une unité de commande de température ;
   c) une surface sur laquelle tous les n-mères ($4^n$) possibles de sondes oligonucléotidiques d'une longueur fixe n sont apposés ; et
   d) un détecteur servant à déterminer la masse des molécules d'acide nucléique liées sur la surface.

**19.** Dispositif selon la revendication 18,
**caractérisé en ce que** la surface lie des molécules d'acide nucléique de manière non spécifique et/ou d'affinité similaire.

**20.** Dispositif selon la revendication 18,
**caractérisé en ce que** la liaison de molécules d'acide nucléique amplifiées est privilégiée par rapport à la liaison d'amorces oligonucléotidiques sur la surface.

**21.** Dispositif selon l'une des revendications 18 à 20,
**caractérisé en ce que** la surface ne lie pas de triphosphates désoxyribonucléotidiques.

**22.** Dispositif selon l'une des revendications 18 à 21,
**caractérisé en ce que** des points de liaison pour les molécules d'acide nucléique sont réparties uniformément sur la surface.

**23.** Dispositif selon la revendication 18,
**caractérisé en ce que** toutes les sondes oligonucléotidiques disposées sur la surface présentent la même longueur.

**24.** Dispositif selon l'une des revendications 18 à 23,
**caractérisé en ce que** la surface comporte du verre silanisé.

**25.** Dispositif selon l'une des revendications 18 à 24,
**caractérisé en ce que** la surface présente une épaisseur adéquate pour l'interférométrie, préférentiellement comprise entre 300 nm et 700 nm, tout préférentiellement comprise entre 400 nm et 600 nm, et le plus préférentiellement de quelque 500 nm.

**26.** Dispositif selon l'une des revendications 18 à 25,
**caractérisé en ce que** le nombre de points de liaison pour les molécules d'acide nucléique sur la surface est supérieur au nombre de molécules d'acide nucléique.

**27.** Dispositif selon l'une des revendications 18 à 26,
**caractérisé en ce que** le rapport de la surface au volume de la chambre est de $10^{-1}$ mm$^2$/$\mu$l à $10^{-3}$ mm$^2$/$\mu$l, préférentiellement de $5 \times 10^{-2}$ mm$^2$/$\mu$l à $5 \times 10^{-3}$ mm$^2$/$\mu$l et tout préférentiellement de quelque 0,02 mm$^2$/$\mu$l.

**28.** Dispositif selon l'une des revendications 18 à 27,
**caractérisé en ce que** le dispositif comporte en outre un calculateur de commande qui enregistre les données de mesure et contrôle la commande de température ainsi que le détecteur.

**29.** Dispositif selon l'une des revendications 18 à 28,
**caractérisé en ce que** le dispositif comporte en outre une entrée pour épreuves et une sortie pour épreuves.

**30.** Dispositif selon l'une des revendications 18 à 29,
**caractérisé en ce que** le détecteur est sélectionné dans le groupe constitué d'un interféromètre en lumière blanche, d'un capteur à ondes de surface, d'une microbalance, d'un capteur de dissipation par microbalance à cristal de quartz et d'un capteur de détection de résonance plasmonique de surface.

**31.** Utilisation du dispositif selon l'une des revendications 18 à 30 en vue de réaliser le procédé selon l'une des revendications 1 à 17.

Abb. 1

Abb. 2

Abb. 3

Abb. 4

Abb. 5

Abb. 6

Abb. 7

Abb. 8

Abb. 9

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4683195 A
- US 4683202 A
- US 4965188 A
- EP 0502588 A
- WO 9729210 A
- US 5716784 A
- DE 19741714
- WO 0102094 A

**In der Beschreibung aufgeführte Nicht-Patentliteratur**

- **M.J. McPherson ; D.D. Hames ; G.R. Taylor.** PCR 1 und 2: A practical Approach. IRL Press at Oxford University Press, 1995
- **F. Lottspeich ; H. Zorbas.** Bioanalytik. Spektrum Akademischer Verlag, 1998
- **N. Barie ; M. Rapp ; H. J. Ache.** UV crosslinked polysiloxanes as new coating materials for SAW devices with high long-term stability. *Sensors and Actuators B,* 1998, vol. 46, 97-103
- **W. Nebe.** Analytische Interferometrie. 1970
- **G. Gauglitz ; A. Brecht ; G. Kraus ; W. Nahm.** *Chemical and biochemical sensors based on interferometry at thin (multi-)layers; Sensors and Actuators B,* 1993, vol. 11, 21-27
- **I. Stemmler ; A. Brecht ; G. Gauglitz.** Compact SPR-transducers with spectral readout for biosensing applications. *Sens. Actuators B,* 1999, vol. 54, 98-105
- **V. Chan et al.** Effect of Hydrophobicity and Electrostatics on Adsorption and Surface Diffusion of DNA Oligonucleotides at Liquid/Solid Interfaces. *Journal of Colloid and Interface Science,* 1998, vol. 203, 197-207
- **U. Maskos ; E.M. Southern.** *Nucleic Acid Research,* 1992, vol. 20, 1679
- **M.J. Gait.** Oligonucleotide Synthesis: A practial Approach. IRL Press at Oxford University Press, 1990
- **C. J. Brinker ; G. W. Scherer.** Sol-gel science : The physics and chemistry of sol-gel processing. Academic Press, 1990